# EUROPÄISCHE PATENTSCHRIFT

(11) **EP 2 496 309 B1**
(45) Veröffentlichungstag und Bekanntmachung des Hinweises auf die Patenterteilung: **10.10.2018**
(21) Anmeldenummer: 10744532.2
(22) Anmeldetag: 13.08.2010
(51) Int. Cl.: A61K 8/46, A61K 8/37, A61K 8/40, A61K 8/895, A61Q 17/04

(54) **CETEARYLSULFOSUCCINATE ENTHALTENDE KOSMETISCHE ZUBEREITUNG MIT FLÜSSIGEN UV-FILTERN**
COSMETIC PREPARATION CONTAINING CETEARYL SULFOCSUCCINATES AND COMPRISING LIQUID UV FILTERS
PRÉPARATION COSMÉTIQUE CONTENANT DES CÉTÉARYLSULFOSUCCINATES AINSI QUE DES FILTRES UV LIQUIDES

(30) Priorität: 07.10.2009 DE 102009048556
(43) Veröffentlichungstag der Anmeldung: 12.09.2012
(73) Patentinhaber: BASF SE, 67056 Ludwigshafen am Rhein (DE)
(72) Erfinder: SKUBSCH, Kerstin, 25497 Prisdorf (DE); TESCH, Mirko, 22303 Hamburg (DE); KOEHLER, Manuela, 22147 Hamburg (DE); MEYER, Christiane, 20357 Hamburg (DE)
(74) Vertreter: BASF IP Association
(86) Internationale Anmeldenummer: PCT/EP2010/004991
(87) Internationale Veröffentlichungsnummer: WO 2011/042088

(56) Entgegenhaltungen:
- EP-A1- 2 179 986
- EP-A2- 0 965 325
- WO-A1-2010/046048
- US-A1- 2008 188 574

## Beschreibung

Die vorliegende Erfindung betrifft eine kosmetische Zubereitung enthaltend Cetearylsulfosuccinate und bestimmte UV-Filter.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen. Ein Hauptnachteil UV-Filter enthaltender kosmetischer Zubereitungen ist, insbesondere wenn es sich um Öl-in-Wasser-Emulsionen (O/W-Emulsionen) handelt, deren Wasserfestigkeit. O/W- Emulsionen sind aufgrund ihrer angenehmen Sensorik (wenig fettig, schnell einziehend) bei den Verbrauchern besonders beliebt. Aufgrund der Tatsache, dass bei O/W-Emulsionen die wässrige Phase die äußere Phase bildet, sind diese Produkte besonders leicht mit Wasser von der Haut abwaschbar. Nach dem Stand der Technik ist es daher erforderlich, Sonnenschutzmitteln auf der Basis einer O/W-Emulsion Filmbildner zuzusetzen, um die Wasserfestigkeit zu erhöhen. Filmbildner führen jedoch dazu, dass die Zubereitung einen klebrig-fettigen Eindruck auf der Haut hinterlassen, schwerer in die Haut einziehen und der Haut einen fettigen Glanz verleihen. Nicht zuletzt sind diese Zubereitungen schwerer verteilbar.
Es war daher die Aufgabe der vorliegenden Erfindung, eine O/W-Emulsion zu entwickeln, die eine höhere Wasserfestigkeit aufweist, als herkömmliche O/W-Emulsionen, ohne dass die sensorischen Eigenschaften darunter leiden.
Ein weiterer Nachteil herkömmlicher Sonnenschutzmittel auf der Basis von O/W-Emulsionen besteht in dem Umstand, dass man diesen Zubereitungen eine relativ große Menge an teuren und schwierig einzuarbeitenden UV-Filter enthalten muss, damit die Zubereitungen einen ausreichenden UV-Schutz (SPF) bieten. Es war daher die Aufgabe der vorliegenden Erfindung, eine O/W-Emulsion zu entwickeln, die mit relativ geringen Konzentrationen an UV-Filtern einen hohen Lichtschutzfaktor (SPF) bietet.

Überraschend gelöst werden die Aufgaben durch eine kosmetische Zubereitung gemäß Anspruch 9. Diese Zubereitung enthält
a) Cetearylsulfosuccinate
b) ein oder mehrere bei Raumtemperatur flüssigen UV-Filter sowie
c) ausgewählte Mercocyanine.
Darüber hinaus werden die Aufgaben gelöst durch die Verwendung von Cetearylsulfosuccinaten zur Erhöhung der Wasserfestigkeit kosmetischer Zubereitungen, welche bei Raumtemperatur flüssige UV-Filter enthalten, und
die Verwendung von Cetearylsulfosuccinaten zur Erhöhung des Lichtschutzfaktors (SPF) kosmetischer Zubereitungen, welche bei Raumtemperatur flüssige UV-Filter enthalten.
Als "bei Raumtemperatur flüssige UV-Filter" gelten UV-Filter, die bei einer Temperatur von 20 °C und 1013 mbar flüssig sind. Insbesondere gelten die folgenden mit ihrer INCI angegebenen UV-Filter erfindungsgemäß als "bei Raumtemperatur flüssige UV-Filter: Homosalate, Octocrylen, Ethylhexylmethoxycinnamat, Ethylhexylsalicylat, Dimethicodiethylbenzalmalonat (Polysilicone-15) und Isoamyl p-Methoxycinnamat. Diese UV-Filter gelten als bevorzugte Ausführungsform der vorliegenden Erfindung.

Die Zubereitungen sind einfach und kostengünstig mit den üblichen Herstellungsverfahren herstellbar. Sie weisen ein ausgesprochen angenehmes Hautgefühl auf. Sie sind farb-, lager- und temperaturstabil sowie photostabil.

Die in dieser Beschreibung der Erfindung als mit "erfindungsgemäß", "erfindungsgemäß vorteilhaft" etc. gekennzeichneten Angaben beziehen sich sowohl auf die kosmetische Zubereitung des Anspruchs 9 als auch auf die erfindungsgemäße Verwendung.
Zwar kennt der Stand der Technik die Firmenpublikation "Eumulgin® Prisma, As diverse as the colors oft he light" (2009) der Fima Cognis, doch konnte diese Schrift nicht den Weg zur vorliegenden Erfindung weisen, da sie nicht die erfindungsgemäßen UV-Filter offenbart, keine Aussagen zur Wasserfestigkeit enthält und den Emulgator als besonders geeignet für Zubereitungen beschreibt, die einen hohen Anteil an UV-Filtern enthalten.
Die Erhöhung der Wasserfestigkeit war insbesondere deshalb überraschend, da die erfindungsgemäßen Cetearylsulfosuccinate, die erfindungsgemäß bevorzugt in Form von Dinatriumsalzen eingesetzt werden, selbst eine gut wasserlösliche Substanz darstellen. Eine verbesserte Wasserfestigkeit auch gegenüber Emulgatorsystemen, die nichtionische Emulgatorbestandteile (beispielsweise Glycerylstearat) enthalten, war daher für den Fachmann nicht zu erwarten.
Das erfindungsgemäße Cetearylsulfosuccinat wird erfindungsgemäß vorteilhaft als Dinatriumsalz eingesetzt. Es handelt sich bei diesem Rohstoff vorteilhaft um eine Mischung aus Dinatriumcetylsulfosuccinat: 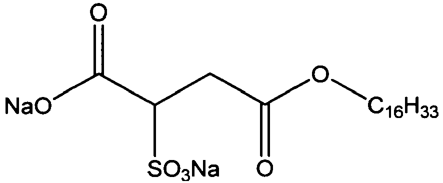 und
Dinatriumstearylsulfosuccinat: 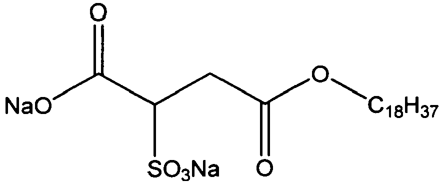 Diese Komponente ist beispielsweise bei der Firma Cognis unter dem Handelsnamen *Eumulgin*® *Prisma* erhältlich.

Es ist erfindungsgemäß vorteilhaft, wenn die kosmetische Zubereitung oder Verwendung dadurch gekennzeichnet ist, dass die Zubereitung Cetearylsulfosuccinate in einer Konzentration von 0,05 bis 2 Gewichts-%, bezogen auf das Gesamtgewicht der Zubereitung, enthält.
Es ist erfindungsgemäß bevorzugt, wenn die kosmetische Zubereitung oder Verwendung dadurch gekennzeichnet ist, dass die Zubereitung Cetearylsulfosuccinate in einer Konzentration von 0,1 bis 1 Gewichts-%, bezogen auf das Gesamtgewicht der Zubereitung, enthält.

Enthält die Zubereitung Homosalate als bei Raumtemperatur flüssigen UV-Filter, so ist es erfindungsgemäß vorteilhaft, wenn die Zubereitung Homosalate in einer Konzentration von 0,5 bis 10 Gewichts-%, bezogen auf das Gesamtgewicht der Zubereitung, enthält.

Enthält die Zubereitung Octocrylen als bei Raumtemperatur flüssigen UV-Filter, so ist es erfindungsgemäß vorteilhaft, wenn die Zubereitung 2-Ethylhexyl-2-cyano-3,3-diphenylacrylat (Octocrylen) in einer Konzentration von 0,5 bis 10 Gewichts-%, bezogen auf das Gesamtgewicht der Zubereitung, enthält.

Enthält die Zubereitung Ethylhexylmethoxycinnamat als bei Raumtemperatur flüssigen UV-Filter, so ist es erfindungsgemäß vorteilhaft, wenn die Zubereitung Ethylhexylmethoxycinnamat in einer Konzentration von 0,2 bis 10 Gewichts-%, bezogen auf das Gesamtgewicht der Zubereitung, enthält.

Enthält die Zubereitung Ethylhexylsalicylat als bei Raumtemperatur flüssigen UV-Filter, so ist es erfindungsgemäß vorteilhaft, wenn die Zubereitung Ethylhexylsalicylat in einer Konzentration von 0,2 bis 5 Gewichts-%, bezogen auf das Gesamtgewicht der Zubereitung, enthält.

Enthält die Zubereitung Dimethicodiethylbenzalmalonat (Polysilicone-15) als bei Raumtemperatur flüssigen UV-Filter, so ist es erfindungsgemäß vorteilhaft, wenn die Zubereitung Dimethicodiethylbenzalmalonat (Polysilicone-15) in einer Konzentration von 0,5 bis 10 Gewichts-%, bezogen auf das Gesamtgewicht der Zubereitung, enthält.

Enthält die Zubereitung Isoamyl p-Methoxycinnamat als bei Raumtemperatur flüssigen UV-Filter, so ist es erfindungsgemäß vorteilhaft, wenn die Zubereitung Isoamyl p-Methoxycinnamat in einer Konzentration von 0,5 bis 10 Gewichts-%, bezogen auf das Gesamtgewicht der Zubereitung, enthält.

Es ist erfindungsgemäß vorteilhaft, wenn die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 2-Hydroxy-4-methoxybenzophenon, 2-Hydroxy-4-methoxy-4'-methylbenzophenon; 2,2'-Dihydroxy-4-methoxybenzophenon; 2-Ethylhexyl-2-hydroxybenzoat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate,enthält.
Die erfindungsgemäß vorteilhaften Merocyanine werden dabei gewählt aus der Gruppe der Verbindungen

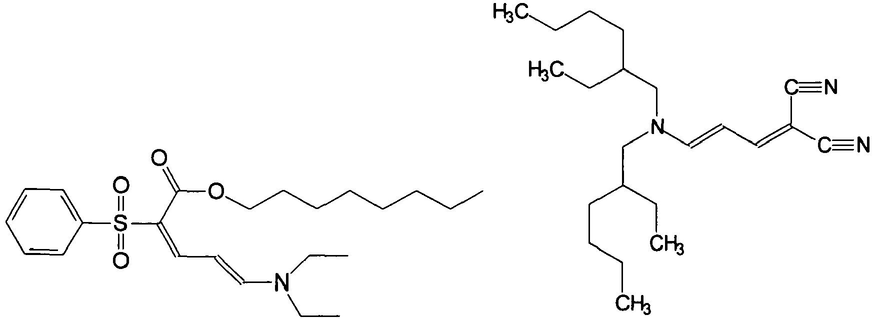

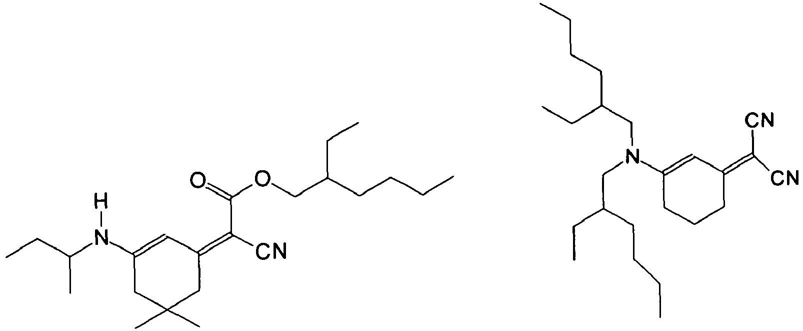

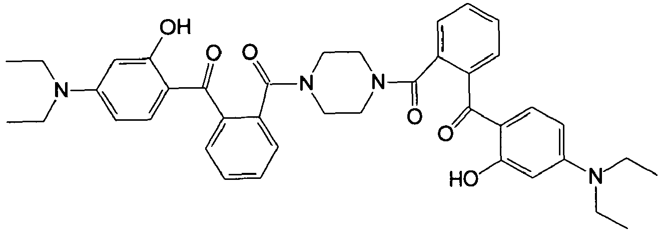

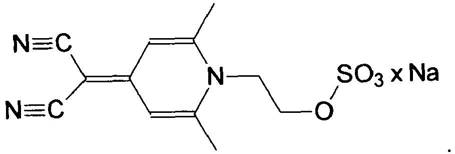

Als erfindungsgemäß vorteilhaftes Piperazinderivat kann dabei die folgende Verbindung eingesetzt werden:

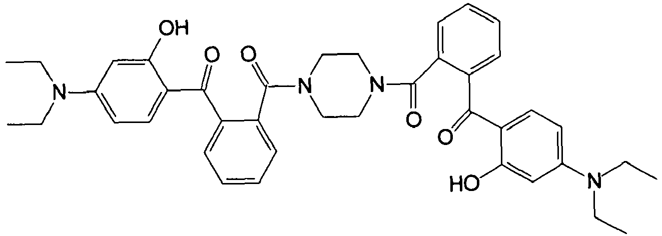

Es ist erfindungsgemäß vorteilhaft, wenn die Zubereitung frei ist von p-Methylbenzylidencampher.
Die Pigmente (Titandioxid, Zinkoxid) können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.
Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₃, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.
Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Es ist erfindungsgemäß vorteilhaft, wenn die Zubereitung einen oder mehrere Parfümstoffe enthält.
Die erfindungsgemäßen Parfümstoffe können beispielsweise gewählt werden aus der Gruppe der Verbindungen 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat, Vanillin, Limonen [5989-27-5], Citral, . Linalool [78-70-6], alpha-Isomethylionon [1335-46-2], Geraniol [106-24-1], Citronellol [106-22-9], [24851-98-7], [18479-58-8], [54464-57-2], [80-54-6], [1222-05,5], [32388-55-9], [105-95-3], [31906-04-4], [8008-57-9], [32210-23-4], [120-57-0], [115-95-7], [101-86-0], [140-11-4], [6259-76-3] und [127-51-5].
Erfindungsgemäß besonders vorteilhafte Ausführungsformen der vorliegenden Erfindung sind dadurch gekennzeichnet, dass die Zubereitung einen oder mehrere Wirkstoffe gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, β-Alanin, Licochalcon A, Hyaluronsäure, Saponine, Dihydroxyaceton; 8-Hexadecen-1,16-dicarbonsäure, Polydocanol, enthält.
Erfindungsgemäß vorteilhafte Ausführungsformen der vorliegenden Erfindung sind dadurch gekennzeichnet, dass die Zubereitung ein oder mehrere Verbindungen gewählt aus der Gruppe der Parabene, Phenoxyethanol, Ethylhexylglycerin, 2-Methylpropan-1,3-diol, Butylenglycol, Propylenglycol , Pentan-1,2-diol, Hexan-1,2-diol, Caprylylglycol enthält. Neben den erfindungsgemäßen bzw. erfindungsgemäß vorteilhaften Bestandteilen, kann die Zubereitung die üblichen Bestandteile wässriger und lipophiler Phasen einer kosmetischen O/W-Emulsion enthalten.

Die Wasserphase der Zubereitungen kann beispielsweise vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether wie Ethylenglykolmonoethyl- oder -monobutylether, Propylenglykolmonomethyl, -monoethyl- oder -monobutylether, Diethylenglykolmonomethyl- oder - monoethylether und analoge Produkte, Polymere, Schaumstabilisatoren, Elektrolyte, Verdickungsmittel, welches oder welche beispielsweise vorteilhaft gewählt werden können aus der Gruppe Siliciumdioxid, Aluminiumsilikate, Polysaccharide bzw. deren Derivate, z. B. Hyaluronsäure, Xanthangummi, Carragenanen, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON),), Ultrathix P 100 von der Firma ISP (INCI: Acrylic Acid/VP Crosspolymer) sowie Aristoflex AVC (INCI: Ammonium Acryloyldimethyltaurate/VP Copolymer) und ARISTOFLEX HMB (Ammonium Acryloyldimethyltaurate/ Beheneth-25 Methacrylate Copolymer).

Die Ölphase der Zubereitung wird beispielsweise vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.
Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).
Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältlich.
Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der Formulierungen einen Gehalt an C₁₂₋₁₅-Alkylbenzoat aufweist oder vollständig aus diesem besteht.
Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise)*.
Auch beliebige Abmischungen solcher Öl- und Wachskomponenten sind vorteilhaft im Sinne der vorliegenden Erfindung einzusetzen.
Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan, Polyolefine, hydrogenierte Polyisobutene, C13-16 Isoparaffin und Isohexadecan. Unter den Polyolefinen sind Polydecene die bevorzugten Substanzen.

Die Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.
Vorteilhaft im Sinne der vorliegenden Erfindung liegen das oder die Siloxanelastomere in Form sphärischer Puder oder in Form von Gelen vor.
Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.
Besonders bevorzugt ist es, wenn das Siloxanelastomer in Kombination mit ölen aus Kohlenwasserstoffen tierischer und/oder pflanzlicher Herkunft, synthetischen Ölen, synthetischen Estern, synthetischen Ethern oder deren Gemischen verwendet wird.

Die Zubereitung kann ein oder mehrere der üblichen Filmbildner zur Erhöhung der Wasserfestigkeit enthalten. Es lassen sich jedoch auch erfindungsgemäß vorteilhafte Zubereitungen ohne dies Filmbildner herstellen.

Erfindungsgemäß vorteilhaft weist die Zubereitung einen pH-Wert von 5 bis 8 auf. Dieser kann durch die herkömmlichen Säuren, Basen und Puffersysteme eingestellt werden.

Zur Anwendung werden die kosmetischen Zubereitungen in der für Kosmetika üblichen Weise auf die Haut und/oder die Haare in ausreichender Menge aufgebracht.

Die Zubereitung kann sprühbar sein.

Erfindungsgemäß vorteilhaft wird die Zubereitung in Kunststoffflaschen aus PE, PP, PET oder Verbundstoffen aufbewahrt.

Der nachfolgende Vergleichsversuch zeigt exemplarisch die erfindungsgemäßen Effekte:

### Vergleichsversuch

Es wurden die folgenden Rezepturen hergestellt und
a) die Wasserfestigkeit mit Hilfe der Kontaktwinkelmessung (siehe "Contact angle measurement-a reliable supportive method for screening water-restistance of ultravioletprotecting products in vivo" Intern. Journal of Cosmetic Science, 2007, 29, 283-291) sowie
b) der in vivo Lichtschutzfaktor (SPF) bestimmt.

| | **Zusammensetzung** | **A** | **B** |
|---|---|---|---|
| Emulgatormischung | Tegin VS + Emulqade F | | 2,65 |
| Emulgator | Disodium Cetearyl Sulfosucinnate | 0,25 | |
| UVA-Filter | Butyl Methoxydibenzoylmethane | 4,5 | 4,5 |
| UVB-Filter | Octocrylene | 4,5 | 4,5 |
| Lichtfiltermischung | Ethylhexyl Methoxycinnamate | 11 | 11 |
| | Phenylbenzimidazole Sulfonic Acid | | |
| | Bis-Ethyl hexyloxyphenol Methoxyphenyl Triazine | | |
| | Diethylhexyl Butamido Triazone | | |
| | Titanium Dioxide | | |
| | Aqua + Trisodium EDTA | | |
| Konservierung | Methylparaben | 9,4 | 9,4 |
| | Phenoxyethanol | | |
| | Alcohol Denat. | | |
| | Glycerin | | |
| Lipophile Phase | Octyldodecanol | 18,7 | 18,7 |
| | Myristyl Myristate | | |
| | Tocopheryl Acetate | | |
| | C12-15 Alkyl Benzoate | | |
| | Butylene Glycol Dicaprylate/Dicaprate | | |
| | Parfum | | |
| | Cetyl Alcohol | | |
| Verdicker | Xanthan Gum | 0,45 | 0,45 |
| | Acrylates/C10-30 Alkyl Acrylate Crosspolymer | | |
| | Tapioca Starch | 1 | 1 |
| Wasserphase | 45%- ige Natronlauqe | q.s. | q.s |
| | Glycerin | 0,9 | 0,9 |
| | VE-Wasser | ad 100 | ad 100 |
| | **in-vivo SPF** | **96** | **67** |
| | **Kontaktwinkel** (je höher desto wasserfester) | **56°** | **23°** |

**Fazit:** Die Zusammensetzung A ist deutlich wasserfester und hat einen höheren Lichtschutzfaktor.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Emulsion** | **1** | **2** | **3** | **4** |
|---|---|---|---|---|
| Disodium Cetearyl Sulfosucinnate | 0,25 | 0,25 | 0,5 | 1 |
| Glyceryl Stearat SE | 1 | | | |
| Natrium Stearoylglutamat | | 0,8 | | |
| Cetearylalkohol | | | 1 | |
| Stearylalkohol | 0,5 | | | |
| Myristylmyristat | 1 | 1,5 | | |
| Acrylates/C₁₀₋₃₀ Alkyl Acrylat Crosspolymer | 0,1 | 0,05 | | |
| Copolymer aus Vinylpyrrolidon und Acrylsäure | | | 0,5 | |
| Carbomer | | | | 0,2 |
| Xanthan Gum | 0,4 | 0,3 | | |
| Natrium Polyacrylat | | | | 0,2 |
| C₁₂₋₁₅ Alkyl Benzoat | | | 4 | |
| Dicaprylyl Caprate | 2 | | | |
| Butylenglykol Dicaprylat/Dicaprat | | 4 | 2 | |
| Isodecyl Neopentanoate | | 5 | | 2 |
| Isopropyl Palmitate | | | 5 | 2 |
| Octyldodecanol | | 2 | | |
| Aluminum Starch Octenylsuccinate | | 1 | | |
| Cyclomethicon | | 3 | 5 | |
| Dimethicon | | | 1 | |
| VP/Hexadecene Copolymer | 0,5 | | 0,2 | |
| Propylenglykol | | 1 | | 5 |
| Glycerin | 9 | 3 | 2 | 3 |
| Octan-1,2-diol | | 5 | 2 | |
| Octocrylen | | 5 | | |
| Octylsalicylat | 5 | 5 | | 2 |
| Ethylhexylmethoxycinnamat | 10 | | | 2 |
| Homosalate | 10 | | 3 | 2 |
| Butyl Methoxydibenzoylmethan | | 5 | | |
| Diethylamino Hydroxybenzoyl Hexyl Benzoat | 5 | | | |
| Bis-Ethylhexyloxyphenol Methoxyphenyl Triazine | | | 1 | |
| Phenylbenzimidazol Sulfonsäure | | 2 | | |
| Tris-biphenyltriazain | | | 2 | |
| Ethylhexyltriazin | | | | 2 |
| Vitamin E Acetat | 0,1 | 0,2 | 0,3 | 0,1 |
| Na₂H₂EDTA | 0,1 | 0,2 | 0,2 | 0,2 |
| Alkohol | 4 | 6 | | |
| Methylparaben | | 0,1 | 0,4 | 0,5 |
| Phenoxyethanol | 0,7 | 1 | 0,5 | |
| Benzethoniumchlorid | 0,05 | | | |
| Piroctone Olamine | | | 0,05 | |
| Ethylhexylglycerin | | | | 1 |

| Natriumhydroxid, Parfüm | q.s. | q.s. | q.s. | q.s. |
|---|---|---|---|---|
| Wasser | ad 100,0 | ad 100,0 | ad 100,0 | ad 100,0 |
| **Emulsion** | **5** | **6** | **7** | **8** |
| Disodium Cetearyl Sulfosucinnate | 0,3 | 0,5 | 0,2 | 0,8 |
| Sucrosepolystearat | | | | 1 |
| Stearylalkohol | 0,5 | | | |
| Cetylalkohol | | 2,2 | | |
| Myristylmyristat | 1 | 1,5 | | |
| Acrylates/C₁₀₋₃₀ Alkyl Acrylat Crosspolymer | 0,1 | 0,05 | | 0,3 |
| Copolymer aus Vinylpyrrolidon und Acrylsäure | | | 1,0 | |
| Xanthan Gum | 0,4 | 0,3 | | |
| Caprylic/Capric Triglyceride | | 3 | | |
| C₁₂₋₁₅ Alkyl Benzoat | | 5 | | 5 |
| Dicaprylyl Caprate | 2 | | | |
| Butylenglykol Dicaprylat/Dicaprat | | 5 | | |
| Diisopropylsebacate | | | | 4 |
| Propylene Glycol Dicaprylate/Dicaprate | 2 | | | |
| Parffinöl | | | | 2 |
| Cyclomethicon | | | 5 | |
| Dimethicon | | 5 | 1 | |
| VP/Hexadecene Copolymer | 0,5 | | 0,2 | |
| Glycerin | 1 | 12 | 5 | 8 |
| Octocrylen | | | 5 | 9 |
| Octylsalicylat | | | | 4 |
| Ethylhexylmethoxycinnamat | | | 5 | |
| Homosalate | | | | 8 |
| Polysilicon-15 | 3 | 4 | | |
| Butyl Methoxydibenzoylmethan | | 3 | | 4,5 |
| Diethylamino Hydroxybenzoyl Hexyl Benzoat | | | 3 | |
| Bis-Ethylhexyloxyphenol Methoxyphenyl Triazine | | 1 | | |
| Phenylbenzimidazol Sulfonsäure | 2 | 0,5 | | |
| Titandioxid | 1 | | | 2 |
| Diemthicone/Polyglycerin-3 Crosspolymer | | 5 | | |
| Dimethicon / Vinyl Dimethicon Crosspolymer | 5 | | 3 | |
| Panthenol | 0,1 | 0,2 | | |
| Na₂H₂EDTA | 0,1 | 0,2 | 0,2 | 0,2 |
| Alkohol | 4 | 6 | | |
| Parfüm | q.s. | q.s. | q.s. | q.s. |
| Konservierungsmittel | q.s. | q.s. | q.s. | q.s. |
| Natriumhydroxid | q.s. | q.s. | q.s. | q.s. |
| Wasser | ad 100,0 | ad 100,0 | ad 100,0 | ad 100,0 |

## Patentansprüche

1. Verwendung von
a. Cetearylsulfosuccinaten zur Erhöhung des Lichtschutzfaktors (SPF) kosmetischer Zubereitungen, welche
b. einen oder mehrere bei Raumtemperatur flüssigen UV-Filter enthalten.

2. Verwendung nach Anspruch 1, **dadurch gekennzeichnet, dass** als flüssige UV-Filter einer oder mehrere der UV-Filter ausgewählt aus Homosalate, Octocrylen, Ethylhexylmethoxycinnamat, Ethylhexylsalicylat, Dimethicodiethylbenzalmalonat (Polysilicone-15) und Isoamyl p- Methoxycinnamat eingesetzt werden.

3. Verwendung nach einem der vorhergehenden Ansprüche, **dadurch gekennzeichnet, dass** die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5- sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4- (1,1,3,3-tetramethylbutyl)-phe-nol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; 2-(4'-Diethylamino-2'-hydoxybenzoyl)-benzoesäurehexylester; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 2-Hydroxy-4-methoxybenzophenon, 2-Hydroxy-4-methoxy-4'- methylbenzophenon; 2,2'-Dihydroxy-4-methoxybenzophenon; 2-Ethylhexyl-2-hydroxybenzoat; 3-(4-(2,2-bis Ethoxycarbonylvinyl)-phenoxy)propenyl)-methoxy-siloxan / Dimethylsiloxan - Copolymer; Dioctylbutylamidotriazon; 2,4-bis-[5-1(dimethylpropyl)-benzoxazol-2-yl-(4-phenyl)-imino]-6-(2-ethylhexyl)-imino-1,3,5-triazin; 4,4',4"-(1,3,5- Triazin-2,4,6-triyltriimino)-tris-benzoesäure-tris(2-ethylhexylester); 2,4-Bis-{[4-(2-elhyl-hexyloxy)-2-hydroxy]-phenyl}-6-(4-methoxyphenyl)-1,3,5-triazin; 2,4,6-Tris-(biphenyl)-1,3,5-triazin; 2,4-Bis-(4'-Di-neopentylaminobenzalmalonat)-6-(4"-butylaminobenzoat)-s-triazin, 4-Di-cyanomethylen-2,6-dimethyl-1,4-dihydropyridin-N-(ethyloxysulfatestersalz), Titandioxid, Zinkoxid, Merocyanine, Piperazinderivate, enthält.

4. Verwendung nach einem der vorhergehenden Ansprüche, **dadurch gekennzeichnet, dass** die Zubereitung einen oder mehrere Parfümstoffe enthält.

5. Verwendung nach einem der vorhergehenden Ansprüche, **dadurch gekennzeichnet, dass** die Zubereitung einen oder mehrere Wirkstoffe enthält, gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym 010, alpha-Glucosylrutin, Carnitin, Carnosin, natürliche und/oder synthetische Isoflavonoide, Glycerylglucose, Kreatin, Kreatinin, Taurin, β-Alanin, Licochalcon A, Hyaluronsäure, Saponine, Dihydroxyaceton; 8-Hexadecen-1,16-dicarbonsäure, Polydocanol.

6. Verwendung nach einem der vorhergehenden Ansprüche, **dadurch gekennzeichnet, dass** die Zubereitung Cetearylsulfosuccinate in Form des Dinatriumsalzes enthält.

7. Verwendung nach einem der vorhergehenden Ansprüche, **dadurch gekennzeichnet, dass** die Zubereitung Cetearylsulfosuccinate in einer Konzentration von 0,05 bis 5 Gewichts-%, bezogen auf das Gesamtgewicht der Zubereitung, enthält.

8. Verwendung von
a. Cetearylsulfosuccinaten zur Erhöhung der Wasserfestigkeit kosmetischer Zubereitungen, welche
b. einen oder mehrere bei Raumtemperatur flüssigen UV-Filter enthalten.

9. Kosmetische Zubereitung enthaltend
a. Cetearylsulfosuccinate sowie
b. ein oder mehrere bei Raumtemperatur flüssigen UV-Filter,
**dadurch gekennzeichnet, dass** die Zubereitung weitere UV-Filter ausgewählt aus der Gruppe der Verbindungen 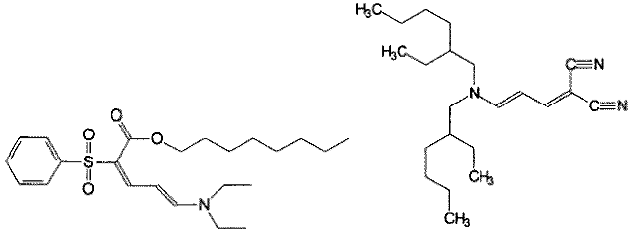 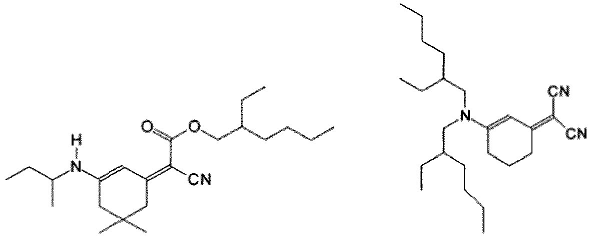 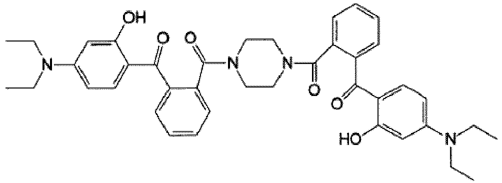 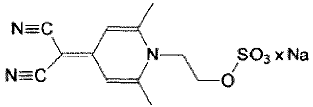 enthält.

10. Kosmetische Zusammensetzung nach Anspruch 9, **dadurch gekennzeichnet, dass** sie als zusätzlichen UV Filter die Verbindung 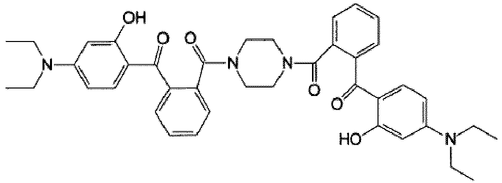 enthält.

## Claims

1. The use of
a. cetearyl sulfosuccinates for increasing the sun protection factor (SPF) of cosmetic preparations which
b. comprise one or more UV filters that are liquid at room temperature.

2. The use as claimed in claim 1, wherein the liquid UV filters used are selected from homosalate, octocrylene, ethylhexyl methoxycinnamate, ethylexyl salicylate, dimethicodiethylbenzalmalonate (polysilicone-15) and isoamyl p-methoxycinnamate.

3. The use as claimed in either of the preceding claims, wherein the preparation comprises one or more UV filters selected from the group of compounds comprising phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidene-methyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; 4-(tert-butyl)-4'-methoxydibenzoylmethane; hexyl 2-(4'-diethylamino-2'-hydoxybenzoyl)benzoate; terephthalidenedicamphorsulfonic acid; 2-ethylhexyl 4-(dimethylamino)benzoate; amyl 4-dimethylamino)benzoate; di(2-ethylhexyl) 4-methoxybenzalmalonate; 2-hydroxy-4-methoxybenzophenone, 2-hydroxy-4-methoxy-4'-methylbenzo-phenone; 2,2'-dihydroxy-4-methoxybenzophenone; 2-ethylhexyl 2-hydroxybenzoate; 3-(4-(2,2-bisethoxy-carbonylvinyl)phenoxy)propenyl)methoxysiloxane / dimethylsiloxane - copolymer; dioctylbutylamidotriazone; 2,4-bis[5-1(dimethylpropyl)benzoxazol-2-yl-(4-phenyl)imino]-6-(2-ethylhexyl)imino-1,3,5-triazine; tris(2-ethylhexyl) 4,4',4''-(1,3,5-triazine-2,4,6-triyltriimino)trisbenzoate; 2,4-bis{ [4-(2-ethylhexyloxy)-2-hydroxy]phenyl}-6-(4-methoxyphenyl)-1,3,5-triazine; 2,4,6-tris(biphenyl)-1,3,5-triazine; 2,4-bis(4'-dineopentyl-aminobenzalmalonate)-6-(4"-butylaminobenzoate)-s-triazine, 4-dicyanomethylene-2,6-dimethyl-1,4-dihydropyridine N-(ethyloxysulfate ester salt), titanium dioxide, zinc oxide, merocyanine; piperazine derivatives.

4. The use as claimed in any of the preceding claims, wherein the preparation comprises one or more perfumes.

5. The use as claimed in any of the preceding claims, wherein the preparation comprises one or more active ingredients selected from the group of compounds comprising tocopheryl acetate, tocopherol, glycyrrhetic acid or salts thereof, alpha-lipoic acid, folic acid, phytoene, D-biotin, coenzyme 010, alpha-glucosylrutin, carnitine, carnosine, natural and/or synthetic isoflavonoids, glycerylglucose, creatine, creatinine, taurine, β-alanine, licochalcone A, hyaluronic acid, saponins, dihydroxyacetone; 8-hexadecene-1,16-dicarboxylic acid, polidocanol.

6. The use as claimed in any of the preceding claims, wherein the preparation comprises cetearyl sulfosuccinates in the form of the disodium salt.

7. The use as claimed in any of the preceding claims, wherein the preparation comprises cetearyl sulfosuccinates at a concentration of 0.05 to 5% by weight, based on the total weight of the preparation.

8. The use of
a. cetearyl sulfosuccinates for increasing the water resistance of cosmetic preparations, which
b. comprises one or more UV filters that are liquid at room temperature.

9. A cosmetic preparation comprising
a. cetearyl sulfosuccinates and
b. one or more UV filters that are liquid at room temperature,
wherein the preparation comprises further UV filters selected from the group of compounds comprising

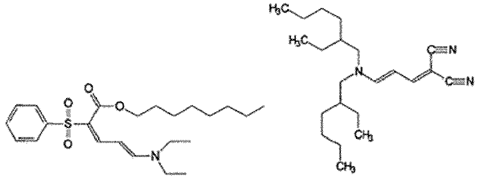

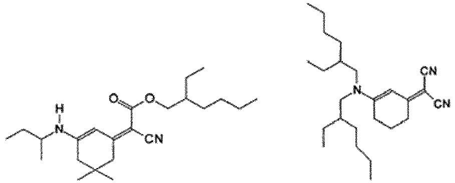

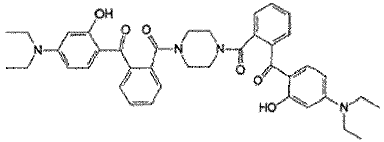

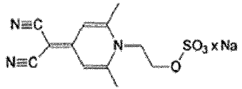

10. The cosmetic composition as claimed in claim 9, wherein as additional UV filter it comprises the compound

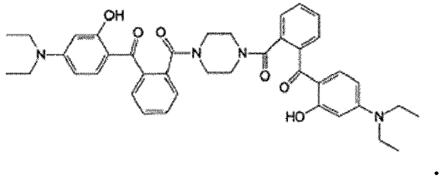

## Revendications

1. Utilisation de
a. sulfosuccinates de cétéaryle pour augmenter le facteur de protection solaire (FPS) de préparations cosmétiques, qui contiennent
b. un ou plusieurs filtres UV liquides à température ambiante.

2. Utilisation selon la revendication 1, **caractérisée en ce qu'**un ou plusieurs filtres UV choisis parmi l'homosalate, l'octocrylène, le méthoxycinnamate d'éthylhexyle, le salicylate d'éthylhexyle, le benzalmalonate de diméthicodiéthyle (polysilicone 15) et le p-méthoxycinnamate d'isoamyle sont utilisés en tant que filtres UV liquides.

3. Utilisation selon l'une quelconque des revendications précédentes, **caractérisée en ce que** la préparation contient un ou plusieurs filtres UV supplémentaires choisis dans le groupe constitué par les composés sels de l'acide phénylène-1,4-bis-(2-benzimidazyl)-3,3'-5,5'-tétrasulfonique ; sels de l'acide 2-phénylbenzimidazol-5-sulfonique ; 1,4-di(2-oxo-10-sulfo-3-bornylidène-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; 4-(tert.-butyl)-4'-méthoxydibenzoylméthane ; ester hexylique de l'acide 2-(4'-diéthylamino-2'-hydroxybenzoyl)-benzoïque ; acide téréphtalidène-dicamphre-sulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; 2-hydroxy-4-méthoxybenzophénone, 2-hydroxy-4-méthoxy-4'-méthylbenzophénone ; 2,2'-dihydroxy-4-méthoxybenzophénone ; 2-hydroxybenzoate de 2-éthylhexyle ; copolymère de 3-(4-(2,2-bis-éthoxycarbonylvinyl)-phénoxy)propényl)-méthoxy-siloxane/diméthylsiloxane ; dioctylbutylamidotriazone ; 2,4-bis-[5-1(diméthylpropyl)-benzoxazol-2-yl-(4-phényl)-imino]-6-(2-éthylhexyl)-imino-1,3,5-triazine ; ester tris(2-éthylhexylique) de l'acide 4,4',4''-(1,3,5-triazin-2,4,6-triyltriimino)-tris-benzoique ; 2,4-bis-{[4-(2-éthyl-hexyloxy)-2-hydroxy]-phényl}-6-(4-méthoxyphényl)-1,3,5-triazine ; 2,4,6-tris-(biphényl)-1,3,5-triazine ; 2,4-bis-(4'-di-néopentylaminobenzalmalonate)-6-(4''-butylaminobenzoate)-s-triazine, sel de l'ester de N-(éthyloxysulfate) de 4-dicyanométhylène-2,6-diméthyl-1,4-dihydropyridine, dioxyde de titane, oxyde de zinc, mérocyanine, dérivés de pipérazine.

4. Utilisation selon l'une quelconque des revendications précédentes, **caractérisée en ce que** la préparation contient un ou plusieurs parfums.

5. Utilisation selon l'une quelconque des revendications précédentes, **caractérisée en ce que** la préparation contient un ou plusieurs agents actifs, choisis dans le groupe constitué par les composés acétate de tocophéryle, tocophérol, acide glycyrrhizique ou ses sels, acide alpha-liponique, acide folique, phytoène, D-biotine, coenzyme 010, alpha-glucosylrutine, carnitine, carnosine, isoflavonoïdes naturels et/ou synthétiques, glycérylglucose, créatine, créatinine, taurine, β-alanine, licochalcone A, acide hyaluronique, saponine, dihydroxyacétone, acide 8-hexadécène-1,16-dicarboxylique, polydocanol.

6. Utilisation selon l'une quelconque des revendications précédentes, **caractérisée en ce que** la préparation contient des sulfosuccinates de cétéaryle sous la forme du sel de disodium.

7. Utilisation selon l'une quelconque des revendications précédentes, **caractérisée en ce que** la préparation contient des sulfosuccinates de cétéaryle en une concentration de 0,05 à 5 % en poids, par rapport au poids total de la préparation.

8. Utilisation de
a. sulfosuccinates de cétéaryle pour augmenter la résistance à l'eau de préparations cosmétiques qui contiennent
b. un ou plusieurs filtres UV liquides à température ambiante.

9. Préparation cosmétique contenant
a. des sulfosuccinates de cétéaryle et
b. un ou plusieurs filtres UV liquides à température ambiante,
**caractérisée en ce que** la préparation contient des filtres UV supplémentaires choisis dans le groupe constitué par les composés

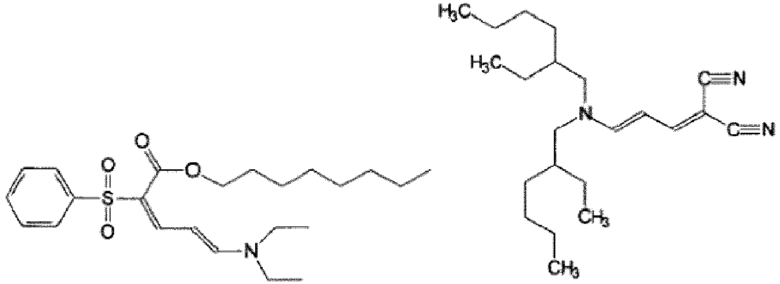

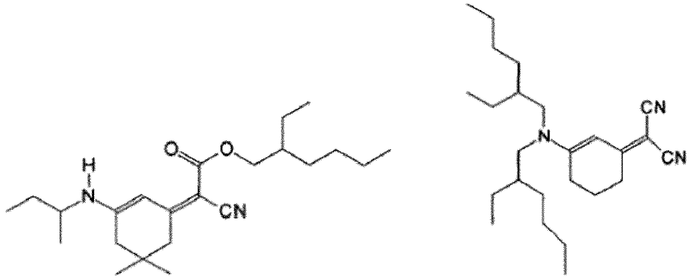

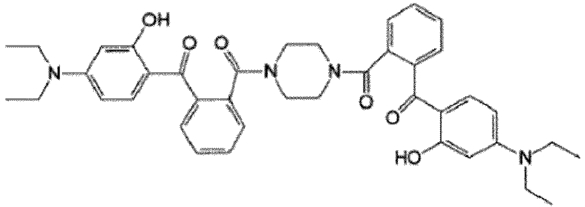

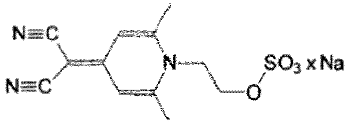

10. Composition cosmétique selon la revendication 9, **caractérisée en ce qu'**elle contient en tant que filtre UV supplémentaire le composé
